# EUROPEAN PATENT APPLICATION

(11) **EP 3 311 814 A1**
(43) Date of publication of application: **25.04.2018**
(21) Application number: 15896415.5
(22) Date of filing: 22.06.2015
(51) Int. Cl.: A61K 31/44, A61K 31/047, A61K 31/70, A61K 9/08, A61K 9/10, A61P 13/00

(54) **PURGATIVE COMPOSITION FOR CLEANSING INTESTINAL TRACT**

(71) Applicant: CTC Bio, Inc., Seoul 138-858 (KR)
(72) Inventor: JEON, Hong Ryeol, Suwon-si Gyeonggi-do 443-751 (KR); LEE, Bong-Sang, Suwon-si Gyeonggi-do 443-757 (KR); KIM, Hyun-Il, Suwon-si Gyeonggi-do 441-822 (KR); LEE, Han-Seung, Namyangju-si Gyeonggi-do 472-501 (KR)
(74) Representative: Nony
(86) International application number: PCT/KR2015/006323
(87) International publication number: WO 2016/208781

(57) **Abstract**

The present invention relates to a purgative composition for cleansing an intestinal tract and, specifically, to a purgative composition for cleansing an intestinal tract, the composition containing 40-60 g of polyethylene glycol, 10-28 g of sorbitol, and 1-10 mg of sodium picosulfate, on the basis of 100 ml of the composition, or 40-60 g of polyethylene glycol, 10-28 g of sorbitol, and 1-15 mg of bisacodyl, on the basis of 100 ml of the composition. The purgative composition according to the present invention has higher drug compliance while showing an excellent intestinal tract cleansing rate. In addition, the purgative composition exhibits a sufficient intestinal tract cleansing effect even when taken together with a small amount of water, thereby relieving the suffering that a patient must take a large amount of medicine when taking a purgative.

## Description

### TECHNICAL FIELD

The present disclosure relates to a purgative composition for cleansing an intestinal tract with high quality of bowel cleansing and high drug compliance, and a method for cleansing an intestinal tract using the same.

### BACKGROUND ART

In bowel cleansing before X-ray examination, endoscopy, radiography or surgery, it is required to empty the intestinal tract as completely as possible. To this end, a commonly used method is evacuation of materials in the form of a suspension from the intestinal tract using water-enemas or hypertonic aqueous solutions including a variety of salts.

Recently, PEG/electrolyte lavage compositions including sodium sulfate, potassium chloride, sodium chloride, sodium bicarbonate, and hydrogen-bond polyethylene glycol (PEG) are used most commonly. However, because these intestinal purgative compositions including PEG/electrolyte require the consumption of about 4L of solution at 2-3 hour intervals (Afridi et al., Gastrointest. Endosc., 1995, 41, 485-489), most of patients suffer from side effects such as discomfort, nausea, cramping pain and vomiting due to large doses (Dipalma et al., Am. J. Gastroenterol., 2003, 98, 2187-2191) and complain that salty taste is unacceptable.

Moreover, to improve the intestinal purgative composition including PEG/electrolyte amounting to 4L, many studies about sodium phosphate composition, magnesium citrate composition, and sodium picosulfate composition have been made, but it is difficult to satisfy patients' safety, improved drug compliance and the bowel cleansing quality.

For example, a sodium phosphate-based intestinal purgative composition is a high osmotic pressure purgative, and it introduces a considerable amount of water into the intestinal tract, and accelerates the movements of the intestines. However, it causes permanent kidney disorders resulting from serious acute phosphate nephropathy, requiring long-term dialysis, and due to this problem, it is high risk medication that is not allowed to prescribe to patients even though it satisfies patients' drug compliance and the quality of bowel cleansing.

Additionally, in the case of an intestinal purgative composition such as magnesium citrate composition and sodium picosulfate composition, patients' drug compliance is high due to small doses, but there is controversy about the quality of bowel cleansing of the composition itself that is significantly lower than conventional intestinal purgative compositions including PEG/electrolyte.

Therefore, there has been a demand for the development of purgatives having a sufficient intestinal tract cleansing effect even at small doses without causing side effects or discomfort.

### DISCLOSURE

### Technical Problem

The present disclosure is directed to providing a purgative for cleansing an intestinal tract with high quality of bowel cleansing and high drug compliance including safety. Furthermore, the present disclosure is directed to providing a method for cleansing an intestinal tract in which a purgative medicine for cleansing an intestinal tract exerts a sufficient effect when it is taken even in a small amount, thereby reducing the suffering in patients who are required to drink medicine in a large amount when taking the purgative.

### Technical Solution

To achieve the object such as the foregoing, the present disclosure provides a purgative composition for cleansing an intestinal tract including polyethylene glycol, sorbitol and sodium picosulfate, or a purgative composition for cleansing an intestinal tract including polyethylene glycol, sorbitol and bisacodyl.

In the study of purgative compositions for cleansing an intestinal tract that satisfy all the drug compliance and the quality of bowel cleansing while not harming patients' safety, the inventors found that a purgative including polyethylene glycol, sorbitol and sodium picosulfate in a specific composition ratio or including polyethylene glycol, sorbitol and bisacodyl in a specific composition ratio has a superior intestinal tract cleansing effect even at small doses with increasing patients' safety and drug compliance, and based on the findings, they completed the present disclosure.

"Purgative" refers to a substance that accelerates the movements of the intestines. Accordingly, the purgative covers diarrhea. For example, the purgative causes mild catharsis effecting diarrhea ("partial purgation") as well as stronger catharsis effecting "complete purgation" or almost complete purgation.

The purgative composition for cleansing an intestinal tract of the present disclosure preferably includes 40 to 60g of polyethylene glycol, 10 to 28g of sorbitol, and 1 to 10mg of sodium picosulfate per 100 ml of the composition in liquid state. Furthermore, the purgative composition for cleansing an intestinal tract of the present disclosure preferably includes 40 to 60g of polyethylene glycol, 10 to 28g of sorbitol, and 1 to 15mg of bisacodyl per 100 ml of the composition in liquid state. When polyethylene glycol is less than 40g per 100 ml of the composition, the intestinal tract cleansing effect as a purgative reduces, and when polyethylene glycol is more than 60g, patients' drug compliance reduces due to the increased dose. When sorbitol is less than 10g per 100 ml of the composition, with the osmotic pressure lower than an isotonic solution, an amount actually used to act as a purgative relative to the dose reduces due to an amount of water absorbed into the body, and when sorbitol is more than 28g, with the osmotic pressure higher than an isotonic solution, it attracts water in the body when it resides in the intestinal tract, affecting water balance in the body. When sodium picosulfate or bisacodyl is less than 1mg per 100ml of the composition, the action as a purgative is insignificant, and when sodium picosulfate is more than 10mg or bisacodyl is more than 15mg, the large intestine is stimulated too much, causing stomachache.

The polyethylene glycol included in the purgative composition may have the number average molecular weight between 1,000 and 10,000. When the molecular weight is less than 1,000, it is prone to absorption into the intestinal mucous membrane, and thus is unsuitable as a purgative. Preferably, polyethylene glycol may have the molecular weight between 2,000 and 6,000, and more preferably, the molecular weight of 3,000 or 4,800.

An osmotic purgative plays a role in increasing the osmotic pressure in the intestines, and as a consequence, promoting the residence of fluids in the intestines. The osmotic purgative that can be used in the purgative composition includes magnesium citrate, magnesium chloride, magnesium hydroxide, magnesium phosphate, magnesium sulfate, magnesium tartrate, sodium phosphate, sodium tartrate, sodium sulfate, potassium tartrate, magnesium oxide, sodium sulfate, or their salt or glycerin, sorbitol, mannitol, lactitol, alcohol sugar, L-sugar, polyethylene glycol or lactulose, and when sorbitol and polyethylene glycol as the osmotic purgative are used together with sodium picosulfate or bisacodyl, the best bowel cleansing quality and drug compliance are provided.

A non-osmotic purgative includes not only stimulant laxatives that directly stimulate the nerve endings in the mucous membrane of the large intestine, but also prokinetic laxatives that stimulate the mobility of the gastrointestinal tract. The non-osmotic purgative that can be used in the purgative composition includes mineral oil, aloe, bisacodyl, sodium picosulfate, casanthranol, cascara, castor oil, dantron, dehydroholic acid, phenolphthalein, sennosides, docusate, bethanachol, colchicines, misoprostol, cisapride, norcisapride, paraffin, rhein or tegaserod, and when sodium picosulfate and/or bisacodyl as the non-osmotic purgative is used together with polyethylene glycol and sorbitol, the best bowel cleansing quality and drug compliance are provided.

A method for preparing the purgative composition may include (S1) putting polyethylene glycol in a container, adding an optimum amount of purified water, and increasing the temperature to 60°C to melt them, (S2) adding an osmotic purgative, a non-osmotic purgative and a sweetening agent, and (S3) adding purified water to adjust the volume and completely melting at 80°C.

The purgative composition may be prepared in a liquid dosage form of a solution such as water and a propylene glycol solution, a suspension and an emulsion, but is not limited thereto. Furthermore, the purgative composition may be prepared with an addition of appropriate sweetening agents, coloring agents, flavoring agents, stabilizing agents and thickening agents.

The sweetening agent included in the purgative composition includes common sugars such as glucose, sucrose, dextrose, fructose and maltose, as well as saccharin, sodium saccharin, xylitol, sorbitol, mannitol, maltitol, lactitol, isomalt, stevioside, erythritol, aspartame, acesulfame potassium and sucralose that have a sweetening effect even in a small amount and fast solubility, but is not limited thereto.

Furthermore, the present disclosure provides a method for cleansing an intestinal tract using the purgative composition. More specifically, the cleansing method includes taking 100 to 200 mL of the purgative composition and 400 to 600mL of water, taking additional 400 to 600 mL of water within 20 minutes, and repeating the administration once more by the same method. Preferably, the cleansing method may include taking 125 to 175 mL of the purgative composition and 450 to 550 mL of water, taking additional 450 to 550 mL of water within 20 minutes, and repeating the administration once more by the same method. Preferably, the cleansing method may include taking 150 mL of the purgative composition and 500 mL of water, taking additional 500 mL of water within 20 minutes, and repeating the administration once more by the same method. Most preferably, the cleansing method may include taking 150 mL of the purgative composition and 350 mL of water within 30 minutes, taking additional 500 mL of water within 30 minutes, resting for a minimum of 1 hour, and repeating the administration once more by the same method. The method for cleansing an intestinal tract using the purgative composition of the present disclosure sufficiently exerts the intestinal tract cleansing effect in the reduced amount of water intake by about 2L.

The present disclosure provides a purgative composition for cleansing an intestinal tract characterized in that 100-200 mL of the purgative composition is diluted with 300-400 mL of water and taken within 20-40 minutes, followed by intake of additional 400-600 mL of water within 20-40 minutes and a rest for 50-70 minutes, and administration is repeated once more by the same method.

Furthermore, the present disclosure provides a purgative composition for cleansing an intestinal tract characterized in that the purgative composition is taken with 5 to 10 vol% of water per volume of the purgative composition. Preferably, the purgative composition may be taken with 5.3 to 8 vol% of water per volume of the purgative composition, and most preferably, 5.5 to 6 vol% of water.

Furthermore, the present disclosure provides a purgative composition for cleansing an intestinal tract characterized in that the purgative composition is taken to the total volume of the purgative composition and water of 3.5 liters or less. Preferably, the purgative composition may be taken to the total volume of the purgative composition and water of 2.5 liters or less, and most preferably, 2 liters or less.

The present disclosure provides a purgative product for cleansing an intestinal tract including a purgative composition for cleansing an intestinal tract including polyethylene glycol, sorbitol and sodium picosulfate, or polyethylene glycol, sorbitol and bisacodyl; and medication instructions stating that the composition be taken with 5 to 10 vol% of water per volume of the composition. The composition included in the purgative product for cleansing an intestinal tract may be preferably taken with 5.3 to 8 vol% of water per volume of the composition, and most preferably, 5.5 to 6 vol% of water.

The composition included in the purgative product for cleansing an intestinal tract may include 40 to 60g of polyethylene glycol, 10 to 28g of sorbitol, and 1 to 10mg of sodium picosulfate per 100 ml of the composition in a liquid dosage form, or may include 40 to 60g of polyethylene glycol, 10 to 28g of sorbitol and 1 to 15mg of bisacodyl per 100 ml of the composition in a liquid dosage form.

The medication instructions included in the purgative product for cleansing an intestinal tract states that 100-200 mL of the composition should be diluted with 300-400 mL of water and taken within 20-40 minutes, followed by intake of additional 400-600 mL of water within 20-40 minutes and a rest for 50-70 minutes, administration should be repeated once more by the same method.

### Advantageous Effects

The purgative composition for cleansing an intestinal tract of the present disclosure has high drug compliance and a superior intestinal tract cleansing effect. Furthermore, the composition exhibits a sufficient effect even when taken together with a small amount of water, thereby reducing the suffering in patients who are required to take medicine in a large amount when taking a purgative.

### BRIEF DESCRIPTION OF THE DRAWINGS

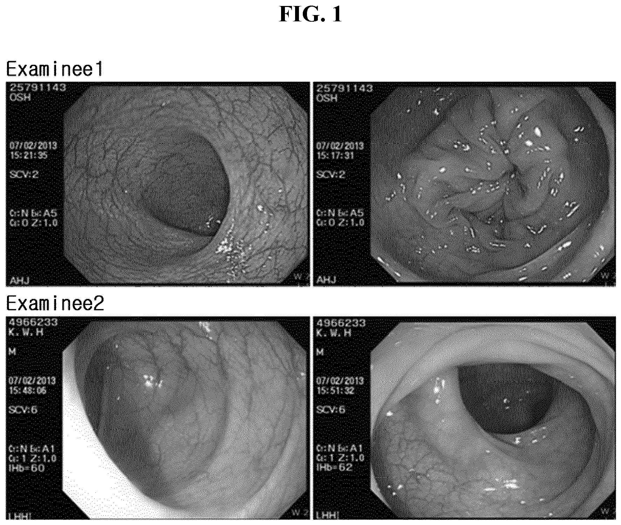

FIG. 1 shows the results of examining the inside of the intestinal tract through endoscopy after taking 100 mL of a purgative composition (undiluted solution) for cleansing an intestinal tract diluted in 500 mL of water, followed by intake of additional 500 mL of water before the next administration, and repeating administration twice more at an interval of 1 hour by the same method.

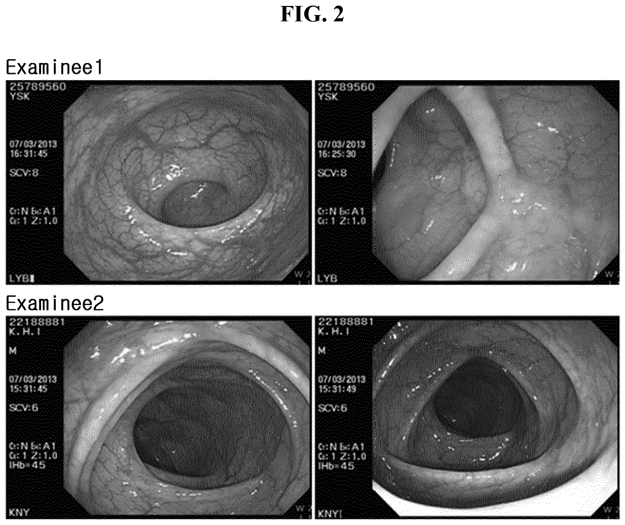

FIG. 2 shows the results of examining the inside of the intestinal tract through endoscopy after taking 100 mL of a purgative composition (undiluted solution) for cleansing an intestinal tract, followed by intake of 500 mL of water within 20 minutes and then additional 500 mL of water before the next administration, and repeating administration twice more at an interval of 1 hour by the same method.

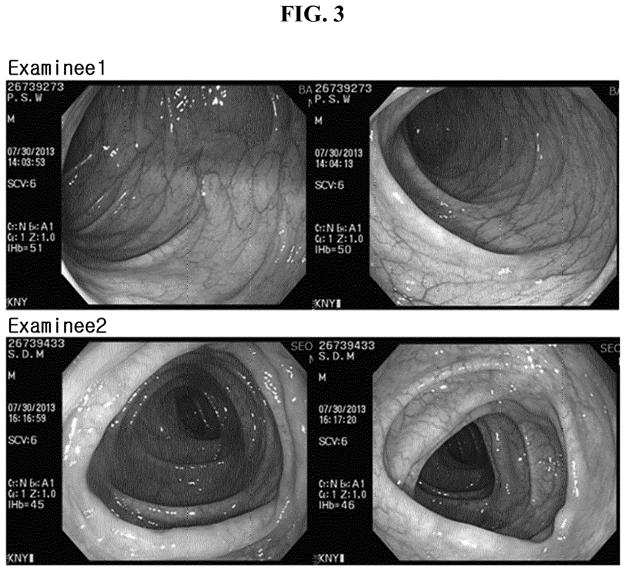

FIG. 3 shows the results of examining the inside of the intestinal tract through endoscopy after taking 150 mL of a purgative composition (undiluted solution) for cleansing an intestinal tract diluted in 500 mL of water, followed by intake of additional 500 mL of water before the next administration, and repeating administration once more at an interval of 1 hour by the same method.

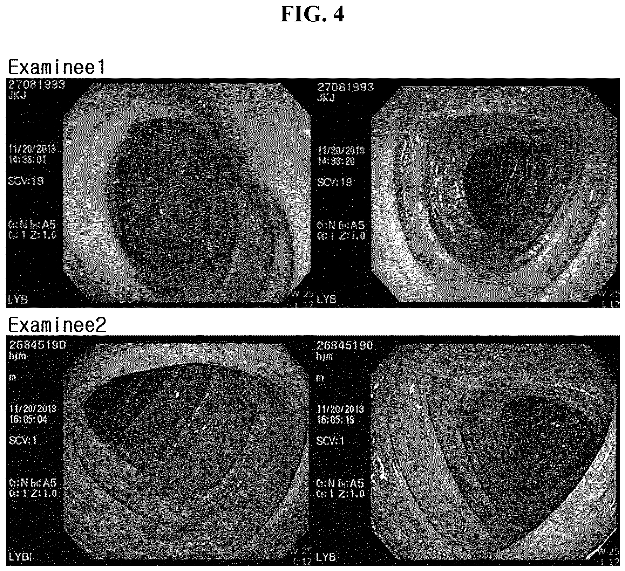

FIG. 4 shows the results of examining the inside of the intestinal tract through endoscopy after taking 150 mL of a purgative composition (undiluted solution) for cleansing an intestinal tract diluted in 350 mL of water within 30 minutes after dilution, followed by intake of additional 500 mL of water within 30 minutes and a rest for a minimum of 1 hour, and repeating administration once more by the same method.

### MODE FOR CARRYING OUT THE INVENTION

Hereinafter, the present disclosure will be described in detail with regard to the embodiments to help the understanding of the present disclosure. However, the embodiments according to the present disclosure may be modified in many other forms, and the scope of the present disclosure should not be interpreted as limiting to the following embodiments. The embodiments of the present disclosure are provided to help those skilled in the art to understand the present disclosure fully and completely.

### [Example 1]

A purgative composition for cleansing an intestinal tract was prepared using the ingredients shown in the following Table 1. To an appropriate container, 150g of polyethylene glycol (number average molecular weight 4800) and then an optimum amount of purified water were added and melted by heating at 60°C, 60 mL of a sorbitol solution (sorbitol 50%), 10 mg of sodium picosulfate and a sweetening agent were added, and purified water was added to make the total volume to 300 mL, and they were completely melted at 80°C.

**[Table 1]**

| Name of raw material | Amount |
|---|---|
| Polyethylene glycol 4800 | 150 g |
| Sorbitol solution (sorbitol 50%) | 60 Ml |
| Sodium picosulfate | 10 mg |
| Sweetening agent | Optimum |
| Purified water | Optimum |
| Total volume | 300 mL |

### [Example 2]

A purgative composition for cleansing an intestinal tract was prepared under the same condition as example 1, except the use of polyethylene glycol with the number average molecular weight of 3000.

### [Example 3]

A purgative composition for cleansing an intestinal tract was prepared using the ingredients shown in the following Table 2. To an appropriate container, 130g of polyethylene glycol (number average molecular weight 4800) and then an optimum amount of purified water were added and melted by heating at 60°C, 90 mL of sorbitol solution (sorbitol 50%), 10 mg of sodium picosulfate and a sweetening agent were added, and purified water was added to make the total volume to 300 mL, and they were completely melted at 80°C.

**[Table 2]**

| Name of raw material | Amount |
|---|---|
| Polyethylene glycol 4800 | 130 g |
| Sorbitol solution (sorbitol 50%) | 90 mL |
| Sodium picosulfate | 10 mg |
| Sweetening agent | Optimum |
| Purified water | Optimum |
| Total volume | 300 mL |

### [Example 4]

A purgative composition for cleansing an intestinal tract was prepared using the ingredients shown in the following Table 3. To an appropriate container, 130g of polyethylene glycol (number average molecular weight 4800) and then an optimum amount of purified water were added and melted by heating at 60°C, 90 mL of sorbitol solution (sorbitol 50%), 15 mg of bisacodyl and a sweetening agent were added, and purified water was added to make the total volume to 300 mL, and they were completely melted at 80°C.

**[Table 3]**

| Name of raw material | Amount |
|---|---|
| Polyethylene glycol 4800 | 130 g |
| Sorbitol solution (sorbitol 50%) | 90 mL |
| Bisacodyl | 15 mg |
| Sweetening agent | Optimum |
| Purified water | Optimum |
| Total volume | 300 mL |

### [Example 5]

A purgative composition for cleansing an intestinal tract was prepared using the ingredients shown in the following Table 4. To an appropriate container, 150g of polyethylene glycol (number average molecular weight 3350) and then an optimum amount of purified water were added and melted by heating at 60°C, 54.6g of sorbitol powder, 10 mg of sodium picosulfate and a sweetening agent were added, and purified water was added to make the total volume to 300 mL, and they were completely melted at 80°C.

**[Table 4]**

| Name of raw material | Amount |
|---|---|
| Polyethylene glycol 3350 | 150 g |
| Sorbitol powder | 54.6 g |
| Sodium picosulfate | 10 mg |
| Sweetening agent | Optimum |
| Purified water | Optimum |
| Total volume | 300 mL |

### Comparative example 1

A purgative composition for cleansing an intestinal tract was prepared using the ingredients shown in the following Table 5. 236g of polyethylene glycol (number average molecular weight 3350), 2.97g of potassium chloride, 5.86g of sodium chloride, 6.74g of sodium hydrogen carbonate, and 22.74g of sodium sulfate anhydrous were added to an appropriate container, then purified water was added to make the total volume to 4L, and they were completed melted.

**[Table 5]**

| Name of raw material | Amount |
|---|---|
| Polyethylene glycol 3350 | 236 g |
| Potassium chloride | 2.97 g |
| Sodium chloride | 5.86 g |
| Sodium hydrogen carbonate | 6.74 g |
| Sodium sulfate anhydrous | 22.74 g |
| Purified water | Optimum |
| Total volume | 4000 mL |

### Comparative example 2

A purgative composition for cleansing an intestinal tract was prepared using the ingredients shown in the following Table 6. 0.01g of sodium picosulfate, 3.5g of magnesium oxide, 12g of citric acid, 0.3g of sodium hydrogen carbonate and a sweetening agent were added to an appropriate container, then purified water was added to make the total volume to 2L, and they were completely melted.

**[Table 6]**

| Name of raw material | Amount |
|---|---|
| Sodium picosulfate | 0.01g |
| Magnesium oxide | 3.5g |
| Citric acid | 12g |
| Sodium hydrogen carbonate | 0.3g |
| Sweetening agent | Optimum |
| Purified water | Optimum |
| Total volume | 2000mL |

### Comparative example 3

### Saline solution for injection

### <Experimental example 1> Comparison of osmotic pressure

For the purgative compositions for cleansing an intestinal tract of examples 1 to 5 and comparative examples 1 to 3, the osmotic pressure was measured using an osmotic pressure meter and its results are shown in the following Table 7. Examples 1 to 5 were measured in diluted state in view of an amount of water taken together, and comparative example 3 is an isometric solution similar to the osmotic pressure in the body. Furthermore, comparative example 1 and comparative example 2 are a commonly used purgative composition for cleansing an intestinal tract including polyethylene glycol/electrolyte or a commonly used purgative composition for cleansing an intestinal tract including sodium picosulfate.

**[Table 7]**

| | Osmotic pressure | Dose |
|---|---|---|
| Example 1 | 265mOsmol | 2L |
| Example 2 | 267mOsmol | 2L |
| Example 3 | 327mOsmol | 2L |
| Example 4 | 326mOsmol | 2L |
| Example 5 | 267mOsmol | 2L |
| Comparative example 1 | 248mOsmol | 4L |
| Comparative example 2 | 44mOsmol | 2L |
| Comparative example 3 | 304mOsmol | - |

Through the results such as those of Table 7, it was found that the osmotic pressure of examples 1 to 5 was similar to the osmotic pressure of comparative example 3 which is a saline solution, and the osmotic pressure of comparative example 2 was the lowest.

Although comparative example 1 shows a good level of osmotic pressure in view of the dose, it can be seen that patients' convenience in administration is the lowest due to the property of comparative example 1 requiring large doses. Furthermore, because polyethylene glycol is a non-ionic polymer that does not react with an electrolyte, taking into account that the electrolyte is absorbed into the body when taken, it is expected that an amount actually used to act as a purgative relative to the dose of 4L will be lower.

### <Experimental example 2> Comparison of intestinal tract cleansing performance based on water intake amount

After taking 100 mL of the composition of example 1 diluted in 500 mL of water, followed by intake of additional 500 mL of water before the next administration, and repeating administration twice more at an interval of 1 hour by the same method, the inside of the intestinal tract was examined through endoscopy, and its results are shown in FIG. 1.

After taking 100 mL of the composition of example 1, followed by intake of 500 mL of water within 20 minutes and then additional 500 mL of water before the next administration, and repeating administration twice more at an interval of 1 hour by the same method, the inside of the intestinal tract was examined through endoscopy, and its results are shown in FIG. 2.

After taking 150 mL of composition of example 1 diluted in 500 mL of water, followed by intake of additional 500 mL of water before the next administration, and repeating administration once more at an interval of 1 hour by the same method, the inside of the intestinal tract was examined through endoscopy, and its results are shown in FIG. 3.

After taking 150 mL of composition of example 5 diluted with 350 mL of water within 30 minutes after dilution, followed by intake of additional 500 mL of water within 30 minutes and a rest for a minimum of 1 hour, and repeating administration once more by the same method, the inside of the intestinal tract was examined through endoscopy, and its results are shown in FIG. 4.

Through this, it was found that the method for cleansing an intestinal tract using the purgative compositions of examples 1 and 5 has sufficiently good intestinal tract cleansing performance at the total dose that was reduced to 2L of water or 2L of water including the purgative composition.

## Claims

1. A purgative composition for cleansing an intestinal tract comprising polyethylene glycol, sorbitol and sodium picosulfate.

2. The purgative composition for cleansing an intestinal tract according to claim 1, wherein the composition comprises 40 to 60g of polyethylene glycol, 10 to 28g of sorbitol, and 1 to 10mg of sodium picosulfate per 100ml of the composition in a liquid dosage form.

3. A purgative composition for cleansing an intestinal tract comprising polyethylene glycol, sorbitol and bisacodyl.

4. The purgative composition for cleansing an intestinal tract according to claim 3, wherein the composition comprises 40 to 60g of polyethylene glycol, 10 to 28g of sorbitol, and 1 to 15mg of bisacodyl per 100ml of the composition in a liquid dosage form.

5. The purgative composition for cleansing an intestinal tract according to one of claims 1 to 4, wherein the polyethylene glycol has a number average molecular weight between 1,000 and 10,000.

6. The purgative composition for cleansing an intestinal tract according to claim 5, wherein the polyethylene glycol has a number average molecular weight between 3,000 and 4,800.

7. The purgative composition for cleansing an intestinal tract according to one of claims 1 to 4, wherein the composition is prepared in a form of any one of a solution, a suspension, and an emulsion.

8. The purgative composition for cleansing an intestinal tract according to one of claims 1 to 4, wherein 100-200 mL of the composition diluted in 300-400 mL of water is taken within 20-40 minutes after the dilution, followed by intake of additional 400-600 mL of water within 20-40 minutes and a rest for 50-70 minutes, and administration is repeated once more by a same method.

9. The purgative composition for cleansing an intestinal tract according to one of claims 1 to 4, wherein the composition is taken with 5 to 10 vol% of water per volume of the composition.

10. The purgative composition for cleansing an intestinal tract according to claim 9, wherein a total volume of the composition and water taken is 3.5 liters or less.

11. A purgative product for cleansing an intestinal tract comprising:
a purgative composition for cleansing an intestinal tract comprising polyethylene glycol, sorbitol and sodium picosulfate, or polyethylene glycol, sorbitol and bisacodyl; and
medication instructions stating that the composition be taken with 5 to 10 vol% of water per volume of the composition.

12. The purgative product for cleansing an intestinal tract according to claim 11, wherein the composition comprises 40 to 60g of polyethylene glycol, 10 to 28g of sorbitol, and 1 to 10mg of sodium picosulfate per 100 ml of the composition in a liquid dosage form, or the composition comprises 40 to 60g of polyethylene glycol, 10 to 28g of sorbitol, and 1 to 15mg of bisacodyl per 100 ml of the composition in a liquid dosage form.

13. The purgative product for cleansing an intestinal tract according to claim 11, wherein the medication instructions states that 100∼200 mL of the composition diluted in 300∼400 mL of water be taken within 20∼40 minutes after the dilution, followed by intake of additional 400∼600 mL of water within 20∼40 minutes and a rest for 50∼70 minutes, and administration is repeated once more by a same method.
